(19) Europäisches Patentamt
European Patent Office
Office européen des brevets

(11) EP 3 841 088 B1

(12) FASCICULE DE BREVET EUROPEEN

(45) Date de publication et mention de la délivrance du brevet:
**25.03.2026 Bulletin 2026/13**

(21) Numéro de dépôt: **19753105.6**

(22) Date de dépôt: **19.08.2019**

(51) Classification Internationale des Brevets (IPC):
***C07C 403/12*** *(2006.01)*

(52) Classification Coopérative des Brevets (CPC):
**C07C 403/12**

(86) Numéro de dépôt international:
**PCT/EP2019/072084**

(87) Numéro de publication internationale:
**WO 2020/038858 (27.02.2020 Gazette 2020/09)**

(54) **PROCÉDÉ DE SYNTHÈSE DE LA VITAMINE A**

VERFAHREN ZUR SYNTHESE VON VITAMIN A

METHOD FOR VITAMIN A SYNTHESIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.08.2018 FR 1857549**

(43) Date de publication de la demande:
**30.06.2021 Bulletin 2021/26**

(73) Titulaire: **Adisseo France S.A.S.**
**92160 Antony (FR)**

(72) Inventeurs:
- **REY, Patrick**
  **69003 LYON (FR)**
- **HUET, Robert**
  **75015 PARIS (FR)**
- **JOERGER, Jean-Michel**
  **69100 VILLEURBANNE (FR)**
- **HENRYON, Vivien**
  **69007 LYON (FR)**

(74) Mandataire: **Germain Maureau**
**12, rue Boileau**
**69006 Lyon (FR)**

(56) Documents cités:
**EP-A1- 1 031 561**
**EP-B1- 1 031 561**
**WO-A1-2012/175398**
**FR-A1- 2 359 822**

Il est rappelé que: Dans un délai de neuf mois à compter de la publication de la mention de la délivrance du brevet européen au Bulletin européen des brevets, toute personne peut faire opposition à ce brevet auprès de l’Office européen des brevets, conformément au règlement d’exécution. L’opposition n’est réputée formée qu’après le paiement de la taxe d’opposition. (Art. 99(1) Convention sur le brevet européen).

## Description

La présente invention concerne de nouvelles réactions de transformation de composés sesquiterpéniques permettant d'ouvrir une nouvelle voie d'accès à la vitamine A ($C_{20}H_{30}O$), ses précurseurs et ses dérivés.

La synthèse à l'échelle industrielle de la vitamine A est réalisée par diverses méthodes classiques. Entre autres préparations, la vitamine A peut être obtenue par la voie de condensation dite C15 + C5, comme par exemple la réaction dite de Julia qui implique la chimie des sulfones, selon laquelle le vinyl-β-ionol est traité par un anion phénylsulfinate pour conduire à une sulfone en C15 à laquelle est ajouté un bromure d'allyle pour obtenir une sulfone en C20. Celle-ci est ensuite convertie, par élimination, en acétate de vitamine A qui est généralement utilisé en l'état, la vitamine A étant très instable, ou alors saponifié en vitamine A.

D'autres procédés sont aussi utilisés. Ainsi, la vitamine A peut être fabriquée par un procédé de couplage en C15 + C5 via une réaction de Wittig ; ce procédé met toutefois en œuvre des intermédiaires à effets cancérogènes, mutagènes ou toxiques pour la reproduction (CMR), tel que l'acétate en C5, et nécessite une unité de régénération de la phosphine au phosgène qui est un gaz très toxique.

Ainsi, on connait selon le document FR2359822A1, un procédé de synthèse de l'acétate de vitamine A à partir de vinyl-β-ionol consistant à faire réagir un sel de β-ionylidene-éthyl-triphénylphosphonium en solution aqueuse avec l'adéhyde γ-acétoxy-glitique.

Une autre voie d'accès réside dans un procédé de couplage en C6 + C14 au travers de la chimie des alcynes ; elle présente les inconvénients d'engager certaines matières premières, comme l'acétylène, le nButyl-Li, qui présentent des risques HSE (Hygiène - Sécurité - Environnement) évidents et d'impliquer des intermédiaires époxydes peu stables et toxiques.

Ces procédés de synthèse n'ont pas connu de véritables évolutions depuis leur découverte, et à ce jour, il est important de recourir à de nouveaux procédés industriels de synthèse plus sûrs et plus économiques.

L'invention fournit une voie originale de synthèse de la sulfone en C15, à partir de sesquiterpènes, et en particulier le farnésène, ou de dérivés sesquiterpéniques, ouvrant un nouveau paradigme dans la synthèse de la vitamine A. Les composés au départ de cette nouvelle voie sont présents dans la nature, on les trouve dans certaines essences végétales d'où ils peuvent être extraits, ils sont aussi biosynthétisés par des microorganismes, notamment des champignons. La ressource en réactif étant donc inépuisable, l'invention apporte une solution pérenne aux problèmes de coût des procédés classiques et contribue à un réel progrès dans la fabrication de la vitamine A, mais aussi celle d'intermédiaires pour d'autres synthèses.

Un des objets de l'invention est un procédé de préparation d'un composé de formule (I)

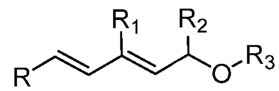

(I)

dans laquelle

R1 est choisi parmi H et les alkyles,
R2 est choisi parmi H, les alkyles, OR' où R' est choisi parmi les alkyles, les silyles, CO-alkyle,
R3 est choisi parmi les groupements acyle de type CO(R") et les groupements suivants CO(OR"), CO(NR"R'''), PO(OR")(OR'''), PO(OR")(R''') où R" et R''', indépendamment l'un de l'autre, sont choisis parmi H et les alkyles ; à titre d'exemple R3 est $CO(CH_3)$ ou $CO(CH_2CH_3)$,
R représente un groupe C(R4)=C(R5)(R6) où R4, R5 et R6, indépendamment les uns des autres, sont choisis parmi H, les alkyles et les alcényles, linéaires ou cycliques, les aryles, les alkylaryles, ou R4 et R5 forment ensemble un cycle, saturé ou insaturé, substitué ou non substitué ;
à titre d'exemple, R représente :

la partie de structure de l'atome de carbone en 1 à l'atome de carbone en 10 d'un rétinoïde, illustrée ci-dessous

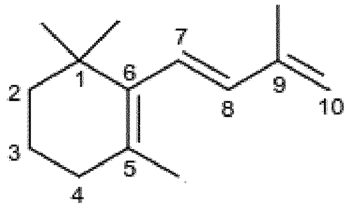

ou la partie de structure de l'atome de carbone en 1 à l'atome de carbone en 10 d'un 7,8-dihydro-rétinoïde, illustrée ci-dessous :

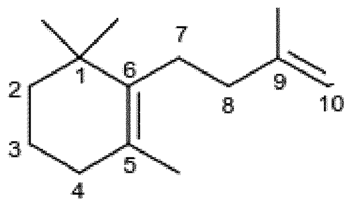

ou la partie de structure de l'atome de carbone en 1 à l'atome de carbone en 6 d'un rétinoïde, illustrée ci-dessous :

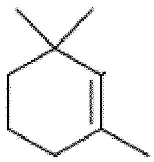
,

ledit composé de formule (I) étant obtenu par réaction d'un composé de formule (II)

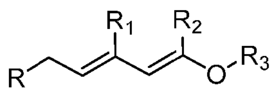
(II)

dans laquelle, R, R1, R2 et R3 ont la définition ci-dessus,
en présence d'une base forte ou en présence d'un catalyseur métallique.

Un autre des objets de l'invention qui constitue un pivot de l'invention, est un procédé monotope d'obtention d'un composé (I) ci-dessus, à partir d'un composé de formule (III)

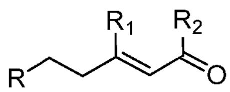
(III)

dans laquelle

R, R1 et R2 ont la définition ci-dessus, ledit procédé comprenant la formation du composé de formule (II)

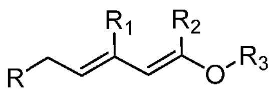
(II)

dans laquelle, R, R1, R2 et R3 ont la définition ci-dessus, à partir dudit composé (III) ci-dessus et la formation du composé (I) selon le procédé d'isomérisation décrit ci-dessus à partir du composé (II). Avantageusement le composé (II) n'est pas isolé. Ce procédé monotope d'acylation/isomérisation représente un réel progrès dans la synthèse de la vitamine A, ses précurseurs et ses dérivés.

Avant d'aborder l'invention plus en détails, les définitions de termes employés dans le présent texte sont ci-après données.

Toute référence à un composé insaturé s'étend aux isomères de ce composé, en particulier à ses régioisomères et ses stéréoisomères.

A titre d'exemple, le terme farnésène inclut les régioisomères alpha et beta du farnésène, ainsi que les stéréoisomères de chacun d'eux, comme illustré ci-dessous :

- L'$\alpha$-farnésène (3,7,11-triméthyl-1,3,6,10-dodécatetraène) ayant la formule suivante

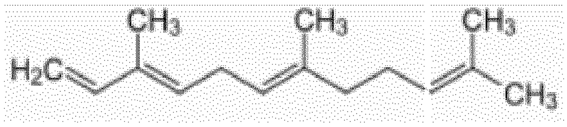

qui peut exister sous la forme des 4 isomères suivants (3E, 6E), (3E, 6Z), (3Z, 6Z) et (3Z, 6E), et

- le β-farnésène (7,11-diméthyl-3-méthylène-1,6,10-dodécatriène) ayant la formule suivante

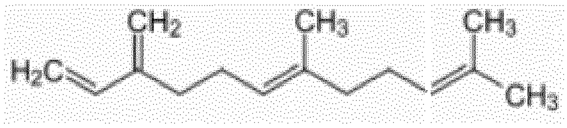

qui peut exister sous la forme des 2 isomères suivants (6E) et (6Z).

Cette définition s'applique notamment au farnésal, au déhydro-farnésal, au farnésol, au rétinal, au dihydrorétinal, leurs acétates et leurs acétates d'énol, dont les appellations couvrent tous leurs isomères respectifs.

Selon l'invention, on entend par un groupe alkyle, une chaîne hydrocarbonée saturée, monovalente, linéaire, cyclique et/ou ramifiée, comportant de 1 à 20 atomes de carbone, de préférence de 1 à 6 atomes de carbone, dont des éléments représentatifs sont par exemple les suivants : les groupes méthyle, éthyle, n-propyle, iso-propyle, n-butyle, sec-butyle, iso-butyle, tert-butyle, pentyle, hexyle. Par un groupe alkyle à chaîne hydrocarbonée cyclique, on entend une chaîne hydrocarbonée saturée, monovalente, comportant de 3 à 20 atomes de carbone, de préférence de 3 à 7 atomes de carbone, et un ou plusieurs cycles. Des éléments représentatifs sont par exemple les suivants : les groupes cyclopropyle, cyclobutyle, cyclopentyle, cyclohexyle, cycloheptyle, norbornyle.

Par un groupe alcényle, on entend une chaîne hydrocarbonée, monovalente, linéaire, cyclique et/ou ramifiée, mono- ou poly-insaturée comprenant de 2 à 20 atomes de carbone.

Un groupe silyle est un groupe constitué d'un atome de Si substitué par 3 substituants, identiques ou différents, choisis parmi H et les alkyles, tel que le triméthylsilyle.

Par un groupe aryle selon l'invention, on comprend une chaine hydrocarbonée aromatique, monofonctionnelle, monocyclique ou polycyclique, comprenant de 6 à 20 atomes de carbone. A titre d'exemples, on peut citer les groupes benzyle, naphtyle et biphényle.

Les termes « alkyle » et « aryle » tels que définis ci-dessus conservent la même définition quand ils intègrent le nom d'un groupe, par exemple dans les groupes -CO-alkyle, alkylaryles.

Selon une variante de l'un quelconque des deux procédés ci-dessus, l'invention concerne la préparation d'un composé de formule (IV)

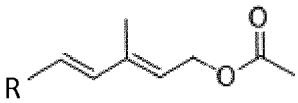

(IV)

dans laquelle R est tel que défini précédemment pour la formule (I),
par réaction d'un composé de formule (V)

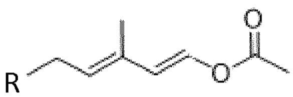

(V)

ou par réaction d'un composé de formule (VI)

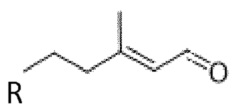

(VI)

en passant par l'intermédiaire de formule (V). Avantageusement le composé (V) n'est pas isolé.

Ainsi, l'un quelconque des composés de formule (I) ou (IV) suivants : l'acétate de vitamine A, le déhydro-β-farnésyl acétate et le déhydro-citral acétate, peut être obtenu :

- selon l’étape d’acylation de l’invention, à partir d’un composé de formule (II) ou (V) choisi parmi le 11,12-dihydrorétinal acétate d’énol, le déhydro-β-farnésyl acétate d’énol et le déhydro-citral acétate d’énol, respectivement, ou
- de préférence selon le procédé d’acylation/isomérisation monotope (ou one-pot) selon l’invention, à partir d’un composé de formule (III) ou (VI) choisi parmi le 11,12-dihydrorétinal, le farnésal et le citral, respectivement, sans isoler l’intermédiaire acétate correspondant.

On a également observé la faisabilité du procédé d’acylation/isomérisation one-pot de l’invention pour obtenir l’acétate de vitamine A à partir du 7,8-dihydro-rétinal. Selon l’invention, il peut être obtenu à partir du déhydro-cyclofarnésyl acétate d’énol, mais de préférence en one-pot à partir du cyclofarnésal sans isoler le déhydro-cyclofarnésyl acétate d’énol.

Dans un mode avantageux de réalisation de l’invention, l’isomérisation du composé (II) ou (V) en composé (I) ou (IV) est réalisée en présence d’une base forte, et par exemple une base forte choisie parmi les phosphazènes tels que $P_2Et$, les aminides telles que le 1,8-diazabicyclo[5.4.0]undec-7-ène (DBU) et les alcoolates tels que le terbutylate de potassium.

L’acétylation du composé (III) ou (VI) en composé (II) ou (V) respectivement, est classiquement effectuée dans des conditions connues de l’homme du métier, par exemple en présence d’anhydride acétique et de pyridine.

Le farnésal est un réactif facilement accessible. Il peut en effet être produit synthétiquement à partir de farnésène, de farnésol, de farnésoate d’éthyle, de nérolidol ou de déhydro-nérolidol selon des procédés connus de l’homme du métier (Tetrahedron Letters 2016, 57, 40, 4496-4499 ; New Journal of Chemistry 2001, 25, 7, 917-929 ; Catal. Comm. 2014, 44, 40-45), mais il peut aussi être isolé d’huiles essentielles, comme celles de lemongrass. Selon une variante originale, le farnésal peut être fabriqué oxydation du farnésène, dans les conditions catalytiques d’un procédé de type Wacker en présence d’au moins un métal précieux, principalement le palladium. Avantageusement, le milieu réactionnel comprend des sels de palladium(II) tels que $PdCl_2$, des sels de cuivre et un agent oxydant. A titre d’exemple, la réaction est conduite en présence de $PdCl_2(CH_2CH_2)$, $CuCl_2$ - $LiMoO_4$.

L’un des intérêts majeurs de l’invention concerna la préparation d’un composé de formule (VII)

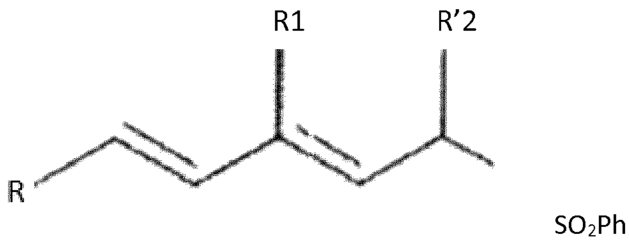

(VII)

dans laquelle
R et R1 ont la définition précédemment donnée pour la formule (I) et R’2 est choisi parmi H et les alkyles.

Ce procédé est aussi un objet de l’invention, et il consiste :

à préparer la sulfone de formule (VII), à partir d’un composé de formule (II) prédécrite, comprenant le procédé d’acylation dudit composé (II) en composé (I), ou
à préparer la sulfone de formule (VII), à partir d’un composé de formule (III) comprenant le procédé d’isomérisation/acylation one-pot dudit composé en composé de formule (I),
lesdits composés (I), (II) et (III) étant tels que définis précédemment.

Dans une variante de l’invention, on prépare la déhydro farnésyle sulfone à partir du farnésal acétate d’énol.

Un des intérêts est de fabriquer la vitamine A à partir de farnésène ou des dérivés de celui-ci, et c’est un autre des objets de l’invention, cette synthèse comprenant au moins l’un quelconque des procédés décrits ci-dessus. Les différents objets de l’invention et leurs applications sont illustrés dans les exemples suivants.

Dans les exemples, les abréviations employées sont ci-après définies :

TT définit le taux de transformation ;
RR définit un rendement sur réactif;
$RR_{isolé}$ définit un rendement sur réactif après isolement ;
$RR_{dosé}$ définit un rendement sur réactif dosé dans un milieu réactionnel.

**Exemple 1: Préparation de l'acétate de vitamine A à partir du 11,12-dihydrorétinal**

Cette préparation comprend deux étapes, une première d'obtention de l'acétate d'énol du 11,12-dihydrorétinal, qui est un composé objet de l'invention, à partir du 11,12-dihydrorétinal, puis une seconde étape mettant en jeu le procédé d'isomérisation de l'invention de l'acétate d'énol du 11,12-dihydrorétinal en acétate de vitamine A.

1.1) Acétylation du 11,12-dihydrorétinal en acétate d'énol et formes isomères

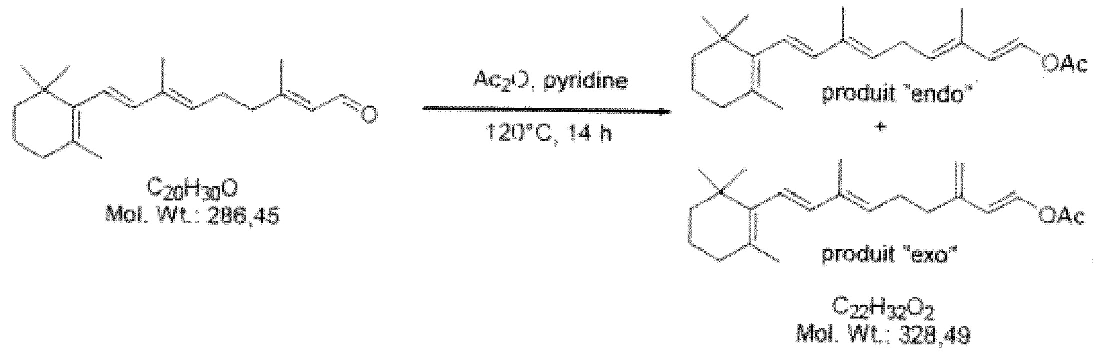


Les conditions opératoires sont les suivantes :
Les réactifs sont introduits sous azote dans l'ordre suivant :

- 11,12-dihydrorétinal (DHR), 2 g (4,47 mmoles)
- DMAP, 1 g (8,10 mmoles)
- Pyridine, 10 g (126 mmoles)
- Anhydride acétique, 10 g (96 mmoles)

Le milieu réactionnel est agité à l'abri de la lumière, à 115°C, pendant 1 h. Après refroidissement à 25°C, le milieu réactionnel est versé dans un mélange constitué de 100 mL d'eau, 100 mL de solution aqueuse saturée de $NaHCO_3$, et 100 mL de cyclohexane. Après séparation, la phase aqueuse est réextraite avec 100 mL de cyclohexane, puis les phases organiques combinées sont lavées par une solution aqueuse saturée de NaCl (300 mL), séchées sur $Na_2SO_4$ et évaporées.

2,01 g d'une huile jaune-orange ont été obtenus.
$TT_{DHR}$ = 100% (CCM)
$RRi_{solé}$ = 57% d'un mélange d'isomères exo/endo (80/20)
Titre (HPLC) = 41% (33% exo + 8% endo).

1.2) Isomérisation du dihydrorétinal acétate d'énol en acétate de vitamine A

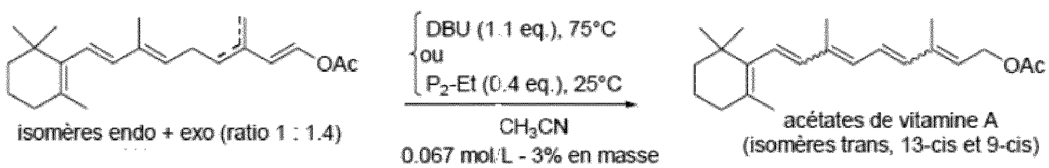


Les conditions opératoires générales sont les suivantes :
Les réactifs ont été introduits sous azote dans un pilulier muni d'une agitation magnétique : acétates d'énols (mélange issu de la réaction 1.2) ci-dessus, 50 mg, 0,076 mmoles), solvant (1,15 mL, sauf DMSO : 2,3 mL), base (tBu0K : 3,6 mg (ajouté en solution dans le cas de DMSO, NMP, isopropanol, et THF) ; hydrotalcite : 25 mg ; KOH : un morceau de 30 mg, titre 85% ; soude aqueuse : 500 µL à 300 g/L). Le milieu réactionnel a été agité à l'abri de la lumière.

Pour le suivi de réaction, chaque prélèvement (50 µL) de phase organique est hydrolysé sur un mélange de 0,5

mL d'eau, 0,5 mL de solution aqueuse saturée de $NaHCO_3$, et 0,5 mL de cyclohexane. Un aliquote de la phase cyclohexane est déposé sur plaque de silice et élué avec un mélange cyclohexane/acétate d'éthyle (90/10).

Différentes conditions ont été testées, les plus représentatives sont indiquées dans le tableau 1.

Tableau 1

| Conditions | RR dosé$_{\text{« trans »}}$ (HPLC, %) |
|---|---|
| $P_2$-ET (0,2 eq.), 25°C, 30 min | 33 |
| DBU (1,1 eq.), 75°C, 120 min | 59 |
| tBuOK (0,4 eq.), 75°C, 30 min | 19 |

**Exemple 2: Préparation de l'acétate de vitamine A à partir du 11,12-dihydrorétinal**

Cet exemple est l'alternative « one-pot » de l'exemple 1 qui comprend le procédé d'acylation/isomérisation selon l'invention.

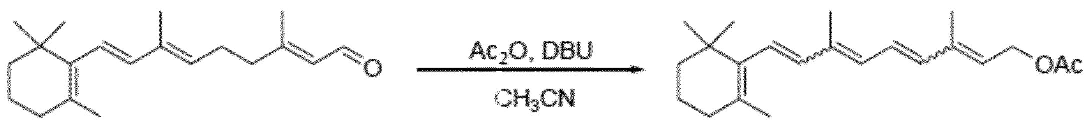


11,12-dihydrorétinal

acétate de vitamine A (trans + 13-cis + 9-cis)

Les conditions opératoires sont les suivantes :
Les réactifs ont été introduits sous azote dans l'ordre suivant :

- Dihydrorétinal, 3,62 g (12 mmoles)
- Acétonitrile, 141,5 g (180 mL)
- $Ac_2O$, 1,86 g (18 mmoles)
- DBU, 9,23 g (60 mmoles)

Le milieu réactionnel (homogène, de couleur jaune foncé) est agité à l'abri de la lumière, à 75°C pendant 10 h. Après refroidissement à 25°C, le milieu réactionnel (homogène, de couleur brun sombre) est versé dans un mélange agité de [cyclohexane (600 mL) + solution aqueuse saturée de bicarbonate de sodium (600 mL) + eau (600 mL)]. La phase aqueuse (pH = 9) est réextraite avec 100 mL de cyclohexane, puis les phases cyclohexane sont combinées, lavées à l'eau (100 mL), séchées ($Na_2SO_4$), et concentrées pour obtenir 3,97 g d'une huile de couleur brun-rouge.

Le brut réactionnel obtenu présente les caractéristiques suivantes :

TT (DHR) = 100% (CCM),

$RR_{\text{isolé}}$ = 77% d'un mélange d'isomères (trans + 13-cis + 9-cis)

Titre (HPLC) = 77% (trans + 13-cis + 9-cis)

Répartition isomérique :

trans/13-cis/9-cis = 75/16/9.

Le brut réactionnel ci-dessus a été cristallisé comme suit.

Une solution de 3,91 g de l'huile précédente dans 3,8 mL de n-heptane est refroidie à -20°C et ensemencée avec des cristaux d'acétate de vitamine A trans (obtenus lors d'une cristallisation précédente dans le n-heptane). Après 4 h à -20°C, une cristallisation massive a lieu. La suspension est ensuite refroidie à 40°C pendant 16 h, puis filtrée. On obtient 2,41 g de cristaux orange (après séchage) et 4,3 mL (3,37 g) d'eaux mères de couleur rouge-brun.

Caractérisation des cristaux :
Rendement de cristallisation de l'isomère trans = 88%

Titre en trans (HPLC) = 81%

Titre (trans + 13-cis + 9-cis, HPLC) = 90%

Répartition isomérique :

trans/13-cis/9-cis = 90/8/2

Caractérisation des eaux mères :

Titre (trans + 13-cis + 9-cis, HPLC) = 23%

Répartition isomérique :

trans/13-cis/9-cis = 37/37/26

**Exemple 3: Préparation de l'acétate de vitamine A à partir du 7,8-dihydrorétinal selon l'alternative « one pot » de l'invention**

Cet exemple est une autre alternative « one-pot » de fabrication de l'acétate de vitamine A qui comprend le procédé d'acylation/isomérisation selon l'invention.

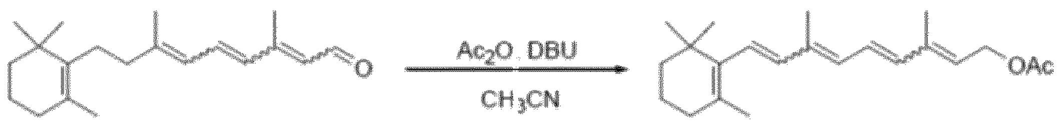


7,8-dihydrorétinènes

Acétate de vitamine A

Les conditions opératoires sont les suivantes :
Les réactifs ont été introduits sous azote à 25°C dans l'ordre suivant :

- 7,8-dihydrorétinal, 0,2 g (0,628 mmoles)
- Acétonitrile, 0,42 mL
- $Ac_2O$, 0,097 g (0,943 mmoles)
- DBU, 0,483 g (3,142 mmoles)

Le milieu réactionnel (homogène, de couleurjaune foncé) est agité à l'abri de la lumière, à 80C. Des prélèvements sont effectués au cours de la réaction pendant 24 heures.

TT (7,8-DHR) = 100% (HPLC)

RR dosé$_{\text{Acétate de Vitamine A}}$ = 4% d'un mélange d'isomères (trans + 13-cis + 9-cis)

après 4 h.
Répartition isomérique :

trans/13-cis/9-cis = 86/10/4

**Exemple 4: Préparation de l'acétate de triène-ol à partir du citral selon l'alternative « one pot » de l'invention**

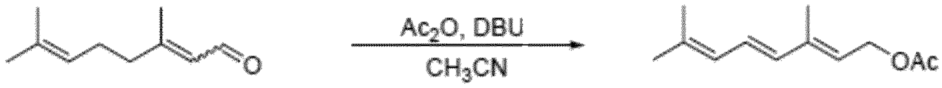


citral

**15**

acétate de triène-ol

Les conditions opératoires sont les suivantes :
Les réactifs ont été introduits sous azote dans l'ordre suivant :

- Citral, 7,7 g (48 mmoles)
- Acétonitrile, 72 mL
- $Ac_2O$, 7,47 g (72 mmoles)
- DBU, 36,5 g (240 mmoles)

Le milieu réactionnel (homogène) est agité à l'abri de la lumière, à 82°C pendant 24 h. Après refroidissement à 25°C, le milieu réactionnel (homogène) est versé dans un mélange agité de [cyclohexane (300 mL) + solution aqueuse saturée de chlorure d'ammonium (300 mL) + eau (300 mL)]. La phase aqueuse (pH = 5) est réextraite avec 100 mL de cyclohexane, puis les phases cyclohexane sont combinées, séchées ($Na_2SO_4$), et concentrées pour obtenir 9,34 g d'une huile de couleur brune.

$$TT\ (DHR) = 100\%\ (CCM)$$

$$RR_{isolé} = 80\%$$

$$Titre\ (RMN\ ^1H) = 80\%$$

**<u>Exemple 5 : Préparation du déhydrofarnésyl acétate à partir de farnésal selon l'alternative « one pot » de l'invention</u>**

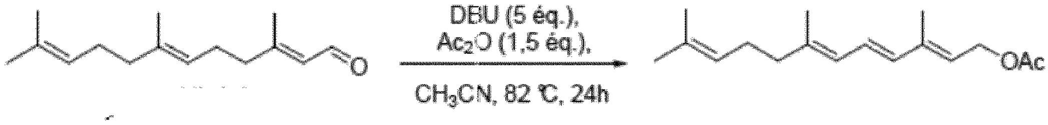


β-farnésal linéaire

<u>déhydrofarnésyl acétate</u>

Les conditions opératoires sont les suivantes :
Dans un tricol de 500 mL équipé d'un barreau d'agitation magnétique et d'un thermomètre, sous atmosphère d'azote, est ajoutée $Ac_2O$ à une solution du farnésal dans $CH_3CN$. Le milieu est agité à 25°C pendant 5 minutes puis le DBU est agité au milieu réactionnel. L'évolution de la composition du milieu réactionnel est suivie par CCM. Après 18h, la conversion du farnésal est complète. Le milieu réactionnel est lavé avec $NH_4Cl$ (2 x50mL), séchée sur $Na_2SO_4$, filtrée, puis concentrée sous pression réduite (40°C, 10 mbar). La purification par chromatographie (SiOH, 120 g, cyclohexane → cyclohexane/AcOEt = 98:2) a permis d'isoler 4,5 g d'une huile orange du déhydrofarnésal acétate.

$$TT\ farnésal = 100\ \%\ (GC)$$

$$RR\ dosé_{déhydrofarnésal\ acétate} = 89\ \%\ (GC)$$

$$RR\ isolé_{déhydrofarnésal\ acétate} = 75\ \%\ (chromatographie)$$

Titre déhydrofarnésal acétate = 90 % (estimation RMN $^1H$)

**<u>Exemple 6: Préparation de la déhydro-farnésylsulfone à partir de déhydrofarnésylacétate obtenu à l'exemple 5</u>**

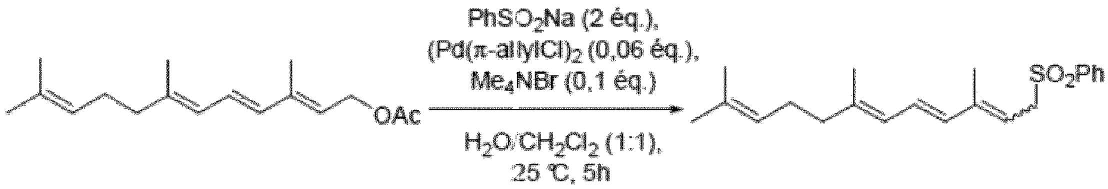


déhydrofarnésyl acétate

<u>déhydro-farnésylsulfone</u>

Les conditions opératoires sont les suivantes :
Dans un tube Schott équipé d'un barreau d'agitation magnétique, sous atmosphère d'azote, est ajoutée une solution de $Pd(p\text{-}allylCl)_2$ (0,028 g, 0,075 mmoles) et 1,1'-Ferrocenediyl-bis(diphenylphosphine (dppf) (0,126 g, 0,21 mmoles) contenu dans $CH_2Cl_2$ (4.4 mL) dégazé ($N_2$) à une solution de $PhSO_2Na$ (1,1 g, 7,57 mmoles) et $Me_4NBr$ (0,15 g, 0,038 mmoles) dans $H_2O$ (12,4 mL) et le déhydrofarnésyl acétate (1,1g, 3,79 mmoles) contenu dans $CH_2Cl_2$ (8,8 mL). L'évolution de la composition du milieu réactionnel est suivie par CCM de la phase organique. Après 5h à 25C, la conversion du déhydrofarnésyl acétate est complète. Le milieu réactionnel est extrait avec $CH_2Cl_2$ (2x20 mL), séché sur $Na_2SO_4$, filtré, puis concentré sous pression réduite (40°C, 10 mbar). La purification par chromatographie (SiOH, 40 g, cyclohexane → cyclohexane/AcoEt = 95:5) a permis d'isoler 1 g d'une huile jaune claire de la <u>déhydro-farnésylsulfone.</u>

$$TT_{\text{déhydrofarnésyl acétate}} = 100\ \%\ (GC)$$

$$RR\ \text{dosé}_{\text{déhydro-farnésylsulfone}} = 76\ \%\ (GC)$$

$$RR\ \text{isolé}_{\text{déhydro-farnésylsulfone}} = 68\ \%\ (\text{chromatographie})$$

Titre déhydro-farnésylsulfone = 90 % (estimation RMN $^1H$)

## Revendications

1. Procédé de préparation d'un composé de formule (I)

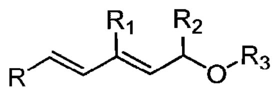

(I)

dans laquelle

R1 est choisi parmi H et les alkyles,
R2 est choisi parmi H, les alkyles, OR' où R' est choisi parmi les alkyles, les silyles, CO-alkyle,
R3 est choisi parmi les groupements acyle de type CO(R"), et les groupements CO(OR"), CO(NR"R‴), PO(OR")(OR‴), PO(OR")(R‴) où R" et R‴, indépendamment l'un de l'autre, sont choisis parmi H et les alkyles,
R représente un groupe C(R4)=C(R5)(R6) où R4, R5 et R6, indépendamment les uns des autres, sont choisis parmi H, les alkyles et les alcényles, linéaires ou cycliques, les aryles, les alkylaryles, ou R4 et R5 forment ensemble un cycle, saturé ou insaturé, substitué ou non substitué,
par réaction d'un composé de formule (II)

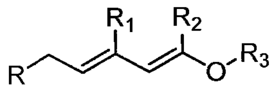

(II)

dans laquelle, R, R1, R2 et R3 ont la définition ci-dessus,
en présence d'une base forte ou en présence d'un catalyseur métallique; dans les formules ci-dessus un groupe alkyle représente une chaîne hydrocarbonée, saturée, monovalente, linéaire, cyclique et/ou ramifiée, comportant de 1 à 20 atomes de carbone, un groupe alcényle représente une chaîne hydrocarbonée, monovalente, linéaire, cyclique et/ ou ramifiée, mono- ou poly-insaturée comprenant de 2 à 20 atomes de carbone, un groupe aryle représente une chaîne hydrocarbonée aromatique, monofonctionnelle, monocyclique ou polycyclique, comprenant de 6 à 20 atomes de carbone et inclut un groupe benzyle et biphényle.

**2.** Procédé monotope de préparation d'un composé de formule (I)

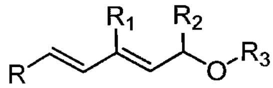

(I)

dans laquelle

R1 est choisi parmi H et les alkyles,
R2 est choisi parmi H, les alkyles, OR' où R' est choisi parmi les alkyles, les silyles, CO-alkyle,
R3 est choisi parmi les groupements acyle de type CO(R"), et les groupements CO(OR"), CO(NR"R‴), PO(OR")(OR‴), PO(OR")(R‴) où R" et R‴, indépendamment l'un de l'autre, sont choisis parmi H et les alkyles,
R représente un groupe C(R4)=C(R5)(R6) où R4, R5 et R6, indépendamment les uns des autres, sont choisis parmi H, les alkyles et les alcényles, linéaires ou cycliques, les aryles, les alkylaryles, ou R4 et R5 forment ensemble un cycle, saturé ou insaturé, substitué ou non substitué,
à partir d'un composé de formule (III)

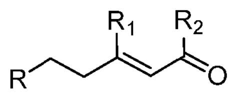

(III)

dans laquelle
R, R1 et R2 ont la définition ci-dessus.
ledit procédé comprenant la formation du composé de formule (II)

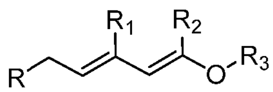

(II)

dans laquelle, R, R1, R2 et R3 ont la définition ci-dessus, et la formation du composé (II) en composé (I) selon le procédé de la revendication 1.

**3.** Procédé selon la revendication 1 ou 2, pour la préparation d'un composé de formule (IV)

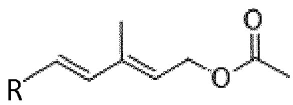

(IV)

dans laquelle R représente un groupe C(R4)=C(R5)(R6) où R4, R5 et R6, indépendamment les uns des autres, sont choisis parmi H, les alkyles et les alcényles, linéaires ou cycliques, les aryles, les alkylaryles, ou R4 et R5 forment ensemble un cycle, saturé ou insaturé, substitué ou non substitué,
par réaction d'un composé de formule (V)

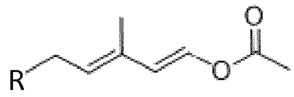

(V)

ou par réaction d'un composé de formule (VI)

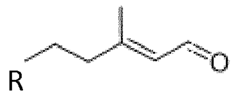

(VI)

en passant par l'intermédiaire de formule (V).

4. Procédé selon la revendication 1 ou 3, pour obtenir un composé de formule (I) ou (IV) choisi parmi l'acétate de vitamine A, le déhydro-farnésyl acétate et le déhydro-citral acétate, **caractérisé en ce que** le composé de formule (II) ou (V) est le 11,12-dihydro-rétinal acétate d'énol, le déhydro-farnésyl acétate d'énol et le déhydro-citral acétate d'énol, respectivement.

5. Procédé selon la revendication 2 ou 3, pour obtenir un composé de formule (I) ou (IV) choisi parmi l'acétate de vitamine A, le déhydro-farnésyl acétate et le déhydro-citral acétate, **caractérisé en ce que** le composé de formule (III) est choisi parmi le 11,12-dihydrorétinal ou le 7,8-dihydrorétinal, le farnésal et le citral, respectivement.

6. Procédé selon l'une quelconque des revendications 1,3 et 4, **caractérisé en ce qu'**il est réalisé en présence d'une base forte, et par exemple une base forte choisie parmi les phosphazènes tels que $P_2Et$, les aminides telles que le 1,8-diazabicyclo[5.4.0]undec-7-ène (DBU) et les alcoolates tels que le terbutylate de potassium.

7. Procédé de préparation d'un composé de formule (VII)

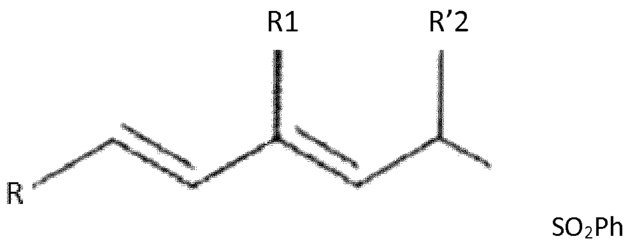

dans laquelle

R1 est choisi parmi H et les alkyles,
R'2 est choisi parmi H et les alkyles,
R représente un groupe C(R4)=C(R5)(R6) où R4, R5 et R6, indépendamment les uns des autres, sont choisis parmi H, les alkyles et les alcényles, linéaires ou cycliques, les aryles, les alkylaryles, ou R4 et R5 forment ensemble un cycle, saturé ou insaturé, substitué ou non substitué
à partir d'un composé de formule (II), comprenant le procédé d'acylation dudit composé (II) en composé (I) selon la revendication 1, ou
à partir d'un composé de formule (III) comprenant le procédé d'isomérisation/acylation one-pot dudit composé (III) en composé de formule (I) selon la revendication 2.

8. Procédé selon la revendication 7, pour la préparation de la déhydro farnésyle sulfone à partir du farnésal acétate d'énol.

9. Procédé de synthèse de la vitamine A à partir de farnésène, **caractérisé en ce qu'**il comprend au moins un procédé selon l'une quelconque des revendications 1 à 8.

10. Acétate d'énol du 11,12-dihydrorétinal en tant que composé intermédiaire.

**Patentansprüche**

1. Verfahren zur Herstellung einer Verbindung mit der Formel (1),

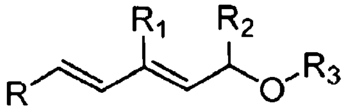

(I)

wobei

R1 aus H und Alkylen ausgewählt ist,
R2 aus H, Alkylen, OR’ ausgewählt ist, wobei R’ aus Alkylen, Silylen, CO-Alkyl ausgewählt ist,
R3 aus den Acylgruppen des Typs CO(R"), und den Gruppen CO(OR"), CO(NR"R‴), PO(OR’")(OR‴), PO(OR‴)(R‴) ausgewählt ist, wobei R" und R‴, unabhängig voneinander, aus H und Alkylen ausgewählt sind,
R für eine Gruppe C(R4)=C(R5)(R6) steht, in der R4, R5 und R6, unabhängig voneinander, aus H, Alkylen und Alkenylen, linear oder zyklisch, Arylen, Alkylarylen ausgewählt sind oder R4 und R5 zusammen einen Kreislauf bilden, gesättigt oder ungesättigt, substituiert oder nicht substituiert,
durch Reaktion einer Verbindung der Formel (II),

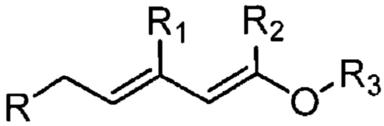

(II)

wobei R, R1, R2 und R3 die obige Definition haben,
in Anwesenheit einer starken Base oder in Anwesenheit eines metallischen Katalysators; in den obigen Formeln stellt eine Alkylgruppe eine gesättigte, monovalente, lineare, zyklische und/oder verzweigte Kohlenwasserstoffkette dar, die 1 bis 20 Kohlenstoffatome aufweist, eine Alkenylgruppe stellt eine monovalente, lineare, zyklische und/oder verzweigte, mono- oder polyungesättigte Kohlenwasserstoffkette dar, die 2 bis 20 Kohlenstoffatome aufweist, eine Arylgruppe stellt eine aromatische, monofunktionelle, monocyklische oder polyzyklische Kohlenwasserstoffkette dar, die 6 bis 20 Kohlenstoffatome umasst eine Benzyl- und Biphenylgruppe enthält.

2. Monotopes Verfahren zur Herstellung einer Verbindung der Formel (1),

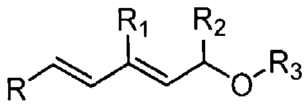

(I)

wobei

R1 aus H und Alkylen ausgewählt ist,
R2 aus H, Alkylen, OR’ ausgewählt ist, wobei R’ aus Alkylen, Silylen, CO-Alkyl ausgewählt ist,
R3 aus den Acylgruppen des Typs CO(R"), und den Gruppen CO(OR"), CO(NR"R‴), PO(OR")(OR‴), PO(OR")(R ‴) ausgewählt ist, wobei R" und R‴, unabhängig voneinander, aus H und Alkylen ausgewählt sind,
R für eine Gruppe C(R4)=C(R5)(R6) steht, in der R4, R5 und R6, unabhängig voneinander, aus H, Alkylen und Alkenylen, linear oder zyklisch, Arylen, Alkylarylen ausgewählt sind oder R4 und R5 zusammen einen Kreislauf bilden, gesättigt oder ungesättigt, substituiert oder nicht substituiert,
aus einer Verbindung der Formel (III),

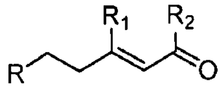

(III)

wobei
R, R1 und R2 die obige Definition haben,
wobei das Verfahren die Bildung der Verbindung mit der Formel (II) umfasst,

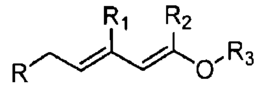 (II)

wobei R, R1, R2 und R3 die oben angegebene Bedeutung, und die Bildung der Verbindung (II) in die Verbindung (1) gemäß dem Verfahren nach Anspruch 1 haben.

**3.** Verfahren nach Anspruch 1 oder 2, zur Herstellung einer Verbindung der Formel (IV),

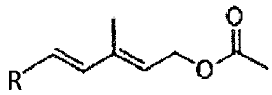 (IV)

wobei R eine Gruppe C(R4)=C(R5)(R6) darstellt, wobei R4, R5 und R6, unabhängig voneinander, aus H, Alkylen und Alkenylen, linear oder zyklisch, Arylen, Alkylarylen ausgewählt sind, oder R4 und R5 zusammen einen Kreislauf bilden, gesättigt oder ungesättigt, substituiert oder nicht substituiert,
durch Reaktion einer Verbindung der Formel (V)

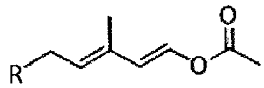 (V)

oder durch Reaktion einer Verbindung der Formel (VI)

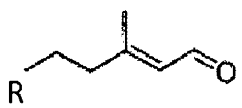 (VI)

über die Formel (V).

**4.** Verfahren nach Anspruch 1 oder 3 zur Herstellung einer Verbindung der Formel (I) oder (IV), die aus Vitamin-A-Acetat, Dehydrofarnesylacetat und Dehydrocitralacetat ausgewählt ist, **dadurch gekennzeichnet, dass** die Verbindung der Formel (II) oder (V) 11,12-Dihydro-Retinalacetat-Enol, Dehydrofarnesylacetat-Enol bzw. Dehydrocitralacetat-Enol ist.

**5.** Verfahren nach Anspruch 2 oder 3 zur Herstellung einer Verbindung der Formel (I) oder (IV), die aus Vitamin-A-Acetat, Dehydrofarnesylacetat und Dehydrocitralacetat ausgewählt ist, **dadurch gekennzeichnet, dass** die Verbindung der Formel (III) aus 11,12-Dihydroretinal oder 7,8-Dihydroretinal, Farnesal und Citral ausgewählt ist.

**6.** Verfahren nach einem der Ansprüche 1, 3 und 4, **dadurch gekennzeichnet, dass** es in Gegenwart einer starken Base durchgeführt wird, beispielsweise einer starken Base, die aus Phosphazenen wie $P_2Et$, Aminiden wie 1,8-Diazabicyclo[5.4.0]undec-7-en (DBU) und Alkoholaten wie Kaliumterbutylat ausgewählt ist.

**7.** Verfahren zur Herstellung einer Verbindung mit der Formel (VII)

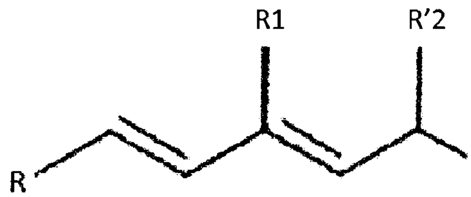

$SO_2Ph$

wobei

R1 aus H und Alkylen ausgewählt ist,

R’2 aus H und Alkylen ausgewählt ist,
R für eine Gruppe C(R4)=C(R5)(R6) steht, in der R4, R5 und R6, unabhängig voneinander, aus H, Alkylen und Alkenylen, linear oder zyklisch, Arylen, Alkylarylen ausgewählt sind, oder R4 und R5 zusammen einen Kreislauf bilden, gesättigt oder ungesättigt, substituiert oder nicht substituiert,
ausgehend von einer Verbindung der Formel (II), umfassend das Verfahren der Acylierung der Verbindung (II) zu einer Verbindung (I) gemäß Anspruch 1, oder
ausgehend von einer Verbindung der Formel (III) umfassend das Ein-Topf-Isomerisierungs-/Acylierungsverfahren der Verbindung (III) zu einer Verbindung der Formel (I) gemäß Anspruch 2.

**8.** Verfahren nach Anspruch 7, zur Herstellung von Farnesyl-Dehydrosulfon aus Enol-Farnesalacetat.

**9.** Verfahren zur Synthese von Vitamin A aus Farnesen, **dadurch gekennzeichnet, dass** es mindestens ein Verfahren gemäß einem der Ansprüche 1 bis 8 umfasst.

**10.** 11,12-dihydroretinales Enolacetat als Zwischenverbindung.

## Claims

**1.** A process for preparing a compound of formula (I)

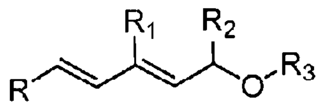

(I)

wherein

R1 is selected from H and alkyls,
R2 is selected from H, alkyls, OR’ where R’ is selected from alkyls, silyls, CO-alkyl,
R3 is selected from acyl groups of the type CO(R"), and CO(OR"),
CO(NR"R‴), PO(OR")(OR‴), PO(OR")(R‴) groups where R" and R‴, independently of each other, are selected from H and alkyls,
R represents a C(R4)=C(R5)(R6) group where R4, R5 and R6, independently of each other, are selected from H, linear or cyclic alkyls and alkenyls, aryls, alkylaryls, or R4 and R5 together form a saturated or unsaturated, substituted or unsubstituted ring,
by reacting a compound of formula (II)

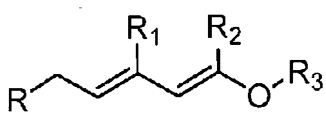

(II)

wherein R, R1, R2 and R3 have the above definition,
in the presence of a strong base or in the presence of a metal catalyst; in the above formulas, an alkyl group represents a saturated, monovalent, linear, cyclic and/or branched hydrocarbon chain comprising 1 to 20 carbon atoms, an alkenyl group represents a monovalent, linear, cyclic and/or branched, mono- or polyunsaturated hydrocarbon chain comprising 2 to 20 carbon atoms, an aryl group represents a monofunctional, monocyclic or polycyclic aromatic hydrocarbon chain comprising 6 to 20 carbon atoms and includes a benzyl and biphenyl group.

**2.** A one-pot process for preparing a compound of formula (I)

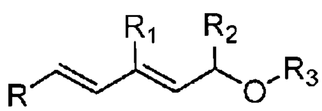

(I)

wherein

R1 is selected from H and alkyls,
R2 is selected from H, alkyls, OR' where R' is selected from alkyls, silyls, CO-alkyl,
R3 is selected from acyl groups of the type CO(R"), and CO(OR"), CO(NR"R‴), PO(OR")(OR‴), PO(OR")(R‴) groups where R" and R‴, independently of each other, are selected from H and alkyls,
R represents a C(R4)=C(R5)(R6) group where R4, R5 and R6, independently of each other, are selected from H, linear or cyclic alkyls and alkenyls, aryls, alkylaryls, or R4 and R5 together form a saturated or unsaturated, substituted or unsubstituted ring,
from a compound of formula (III)

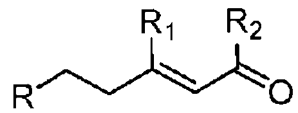

(III)

wherein
R, R1 and R2 have the above definition.
said process comprising the formation of the compound of formula (II)

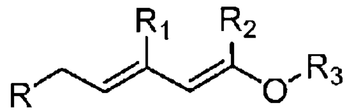

(II)

wherein R, R1, R2 and R3 have the above definition, and the formation of the compound (II) to a compound (I) according to the process of claim 1.

3. The process according to claim 1 or 2, for the preparation of a compound of formula (IV)

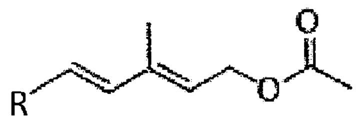

(IV)

wherein R represents a C(R4)=C(R5)(R6) group where R4, R5 and R6, independently of each other, are selected from H, linear or cyclic alkyls and alkenyls, aryls, alkylaryls, or R4 and R5 together form a saturated or unsaturated, substituted or unsubstituted ring,
by reacting a compound of formula (V)

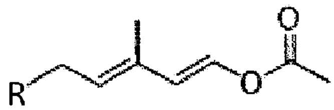

(V)

or by reacting a compound of formula (VI)

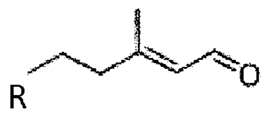

(VI)

via the intermediate of formula (V).

4. The process according to claim 1 or 3, for obtaining a compound of formula (I) or (IV) selected from vitamin A acetate, dehydro-farnesyl acetate and dehydro-citral acetate, **characterized in that** the compound of formula (II) or (V) is 11,12-dihydroretinal enol acetate, dehydro-farnesyl enol acetate and dehydro-citral enol acetate, respectively.

5. The process according to claim 2 or 3, for obtaining a compound of formula (I) or (IV) selected from vitamin A acetate, dehydro-farnesyl acetate and dehydro-citral acetate, **characterized in that** the compound of formula (III) is selected from 11,12-dihydroretinal or 7,8-dihydroretinal, farnesal and citral, respectively.

**6.** The process according to any one of claims 1, 3 and 4, **characterized in that** it is carried out in the presence of a strong base, and for example a strong base selected from phosphazenes such as $P_2Et$, amidines such as 1,8-diazabicyclo [5.4.0]undec-7-ene (DBU) and alkoxides such as potassium tertbutoxide.

**7.** A process for preparing a compound of formula (VII)

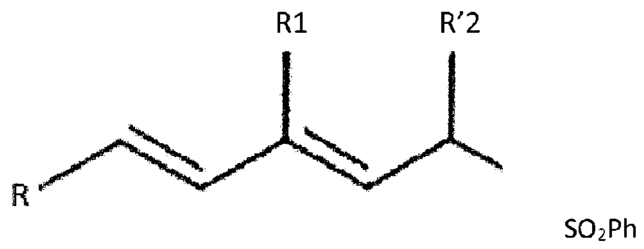

wherein

R1 is selected from H and alkyls,
R'2 is selected from H and alkyls,
R represents a C(R4)=C(R5)(R6) group where R4, R5 and R6, independently of each other, are selected from H, linear or cyclic alkyls and alkenyls, aryls, alkylaryls, or R4 and R5 together form a saturated or unsaturated, substituted or unsubstituted ring
from a compound of formula (II), comprising the process for acylating said compound (II) to a compound (I) according to claim 1, or
from a compound of formula (III) comprising the process of one-pot isomerization/acylation of said compound (III) to a compound of formula (I) according to claim 2.

**8.** The process according to claim 7 for the preparation of dehydro-farnesyl sulfone from farnesal enol acetate.

**9.** A process for synthesizing vitamin A from farnesene, **characterized in that** it comprises at least one process according to any one of claims 1 to 8.

**10.** 11,12-Dihydroretinal enol acetate as an intermediate compound.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2359822 A1


### Littérature non-brevet citée dans la description


- *Tetrahedron Letters*, 2016, vol. 57 (40), 4496-4499
- *New Journal of Chemistry*, 2001, vol. 25 (7), 917-929
- *Catal. Comm.*, 2014, vol. 44, 40-45